# EUROPEAN PATENT SPECIFICATION

(11) **EP 2 678 683 B1**
(45) Date of publication and mention of the grant of the patent: **17.08.2016**
(21) Application number: 12707649.5
(22) Date of filing: 21.02.2012
(51) Int. Cl.: G01N 33/569

(54) **A METHOD FOR THE PROGNOSIS OF PROGRESSION OF THE HIV DISEASE**
VERFAHREN ZUR PROGNOSE DER WEITERENTWICKLUNG DER HIV-ERKRANKUNG
MÉTHODE PERMETTANT DE PRONOSTIQUER L'ÉVOLUTION DE L'INFECTION PAR LE VIH

(30) Priority: 22.02.2011 EP 11305187
(43) Date of publication of application: 01.01.2014
(73) Proprietor: INNAVIRVAX, 91058 Evry (FR); Institut National de la Santé et de la Recherche Médicale, 75013 Paris (FR)
(72) Inventor: MEYER, Laurence, F-94110 Arcueil (FR); VIEILLARD, Vincent, F-75004 Paris (FR); DEBRE, Patrice, F-75006 Paris (FR); CROUZET, Joël, F-92330 Sceaux (FR)
(74) Representative: Nony
(86) International application number: PCT/IB2012/050787
(87) International publication number: WO 2012/114272

(56) References cited:
- WO-A1-2010/040853
- NGO-GIANG-HUONG NICOLE ET AL: "HIV type 1-specific IgG2 antibodies: Markers of helper T cell type 1 response and prognostic marker of long-term nonprogression", AIDS RESEARCH AND HUMAN RETROVIRUSES, vol. 17, no. 15, 10 October 2001 (2001-10-10), pages 1435-1446, XP055004015, ISSN: 0889-2229
- anonymous: "DIAG3S : AN AIDS PROGNOSTIC TEST", , 2011, page 1, XP055001571, Retrieved from the Internet: URL:http://www.innavirvax.fr/RD-projects/m enu-id-4.html [retrieved on 2011-06-28]
- HUGUES FAUSTHER-BOVENDO ET AL: "HIV gp41 Engages gC1qR on CD4+ T Cells to Induce the Expression of an NK Ligand through the PIP3/H2O2 Pathway", PLOS PATHOGENS, vol. 6, no. 7, 1 July 2010 (2010-07-01), page E1000975, XP055001572, DOI: 10.1371/journal.ppat.1000975
- DEBRE PATRICE ET AL: "[Towards a vaccine for HIV infection: role of the gp41 envelope protein].", BULLETIN DE L'ACADÉMIE NATIONALE DE MÉDECINE JAN 2009 LNKD- PUBMED:19718985, vol. 193, no. 1, January 2009 (2009-01), pages 127-136 ; DIS, XP008139380, ISSN: 0001-4079
- VIEILLARD VINCENT ET AL: "Specific adaptive humoral response against a gp41 motif inhibits CD4 T-cell sensitivity to NK lysis during HIV-1 infection", AIDS, LONDON, GB, vol. 20, no. 14, 1 September 2006 (2006-09-01), pages 1795-1804, XP008100308, ISSN: 0269-9370 cited in the application
- ANONYMOUS: 'RESEARCH & DEVELOPMENT PROJECTS' INNAVIRVAX, [Online] 12 September 2009, page 1, XP055208144 Retrieved from the Internet: <URL:http://web.archive.org/web/20090912041 108/http://www.innavirvax.fr/RD-projects/me nu-id-4.html> [retrieved on 2015-08-17]
- VIEILLARD VINCENT ET AL: "NK cytotoxicity against CD4+ T cells during HIV-1 infection: A gp41 peptide induces the expression of an NKp44 ligand", PROCEEDINGS OF THE NATIONAL ACADEMY OF SCIENCES, NATIONAL ACADEMY OF SCIENCES, US, vol. 102, no. 31, 2 August 2005 (2005-08-02), pages 10981-10986, XP002341851, ISSN: 0027-8424, DOI: 10.1073/PNAS.0504315102
- CASADO CONCEPCION ET AL: 'Host and Viral Genetic Correlates of Clinical Definitions of HIV-1 Disease Progression' PLOS ONE vol. 5, no. 6, E11079, June 2010, ISSN: 1932-6203
- VAN DER HELM JANNIE J ET AL: "Characterisation of long-term non-progression of HIV-1 infection after seroconversion: a cohort study.", THE LANCET. HIV OCT 2014, vol. 1, no. 1, October 2014 (2014-10), pages e41-e48, ISSN: 2352-3018

## Description

The present invention relates to the field of prognosis of the HIV disease.

Infection of human beings with an HIV virus is generally diagnosed by immunodetection of anti-HIV antibodies in blood plasma samples. Numerous HIV ELISA test kits are commercially available today. Generally, the known HIV ELISA test kits use polystyrene microwell strips which are pre-coated with a mixture of HIV antigens, which HIV antigens may consist of recombinant HIV antigens expressed in *E. coli,* such as recombinant HIV-1 gp41, gp120 and HIV-2 gp36 glycoproteins.

Once the infection of an individual with an HIV virus has been diagnosed, a follow-up of the progression of the HIV disease shall be performed, including for the purpose of determining when to start an anti-retroviral pharmaceutical treatment (ART). This requires performing an HIV staging method on the said individual.

HIV disease staging and classification systems are critical tools providing clinicians and patients essential information for clinical management. Two major classification systems currently are in use, respectively (i) system the World Health Organization (WHO) Clinical Staging end Disease Classification System and (ii) the US Centers for Disease Control and Prevention (CDC) classification.

The WHO Clinical Staging and Disease Classification System of HIV/AIDS is mainly based on the determination of the occurrence of clinical events without compulsory requiring the results from laboratory testing. The WHO clinical staging system has been widely used in resource-limited countries and has proved pragmatic and useful in facilities at both the first level and the referral level.

The CDC disease staging system assesses the severity of the HIV disease by CD4+ T lymphocyte cell (CD4) counts and by the presence of specific HIV-related conditions. According to the CDC staging system, the definition of AIDS includes all HIV-infected individuals with CD4 counts of less than 200 cells/µl, or a CD4 percentage (over all lymphocytes) of less than 14%, as well as those with certain HIV-related conditions and symptoms. Thus, according to the CDC system, the CD4 count consists of the standard laboratory test for assessing HIV stage and prognosis, and for monitoring progression to AIDS and risk of opportunistic illnesses. The CD4 cell count also guides the physician in formulating differential diagnoses in symptomatic patients, and in deciding about initiating antiretroviral treatment (ART), and beginning prophylaxis for opportunistic infections.

In untreated HIV infection, HIV replication usually produces billions of new HIV copies daily. Plasma HIV RNA (viral load) testing quantifies the HIV viral burden in the plasma. In areas with access to viral load monitoring, the viral load is a standard tool used to monitor treatment response in patients taking ART and, in conjunction with the CD4 cell count, to assess HIV progression (see notably Mellors et al., 1996, Science, Vol. 272(5265): 1167-170).

AIDS is a pandemic disease affecting more than 30 million people throughout the world with more than 2 millions new infected people per year. To date, AIDS remains a fatal disease which has killed more than 2 million people in 2009.

Beside the need for availability for a plurality of distinct therapeutic treatments or for continuously improving existing ARTs, there is also a constant need for a plurality of staging or prognosis methods, so as to provide physicians with precise or complementary tools aimed notably at determining the appropriate time period for starting ART, and helping them to correctly adapt treatments to the disease progression profile, on a case-by-case basis.

### SUMMARY OF THE INVENTION

The present invention relates to an *in vitro* method for the prognosis of progression of an HIV-1 disease in a patient infected with an HIV-1 virus, who has not the rare phenotype of asymptomatic long-term survival, which prognosis is based on the level of antibodies directed against 3S peptide of SEQ ID NO. 2 as a prognostic marker, which prognostic marker is independent from the CD4 count prognosis marker, the said method comprising the steps of :
a) measuring the level of antibodies directed against a 3S peptide of SEQ ID NO. 2 in a sample collected from the said patient,
b) comparing the level of antibodies measured at step a) with a reference value that is indicative of the progression of the HIV-1 disease.

### DESCRIPTION OF THE FIGURES

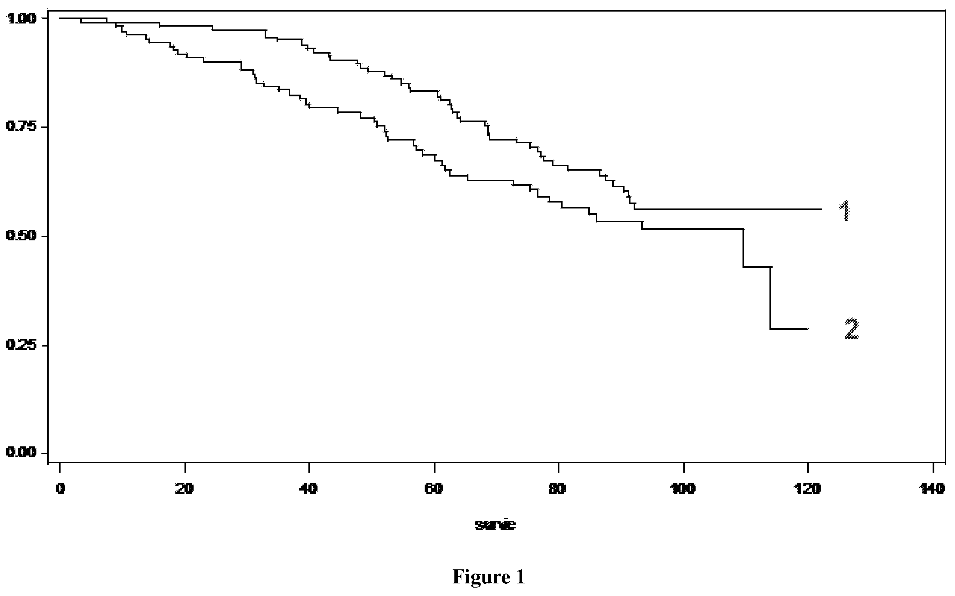

**Figure 1** is a diagram depicting the Kaplan-Meier curves showing the survival time on a 120-months time period of HIV-1-infected patients having a CD4 count above 200 cells/µl and having an anti-3S antibody level respectively above (upper curve n° 1) or below (lower curve n° 2) the predetermined reference value (here, the predetermined reference value, or "cut-off" value, is of 50). Abscissa : time period following the date of HIV-1 infection, as expressed in months. Ordinate : survival distribution (100% =1 Unit).

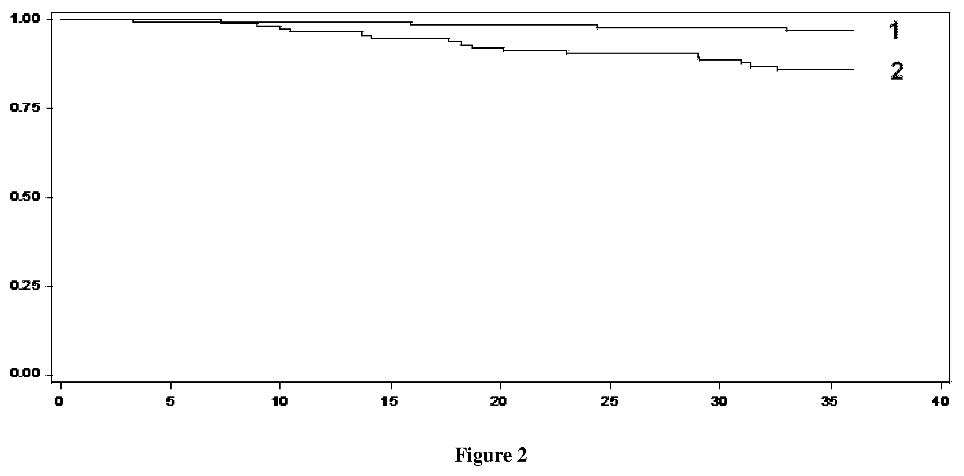

**Figure 2** is a diagram depicting the Kaplan-Meier curves showing the survival time on a 36-months time period of HIV-1-infected patients having a CD4 count above 200 cells/mm³ and having an anti-3S antibody level respectively above (upper curve n° 1) or below (lower curve n° 2) the predetermined reference value (here, the predetermined reference value, or "cut-off" value, is of 50). Abscissa: time period following the date of HIV-1 infection, as expressed in months. Ordinate: survival distribution (100% =1 Unit).

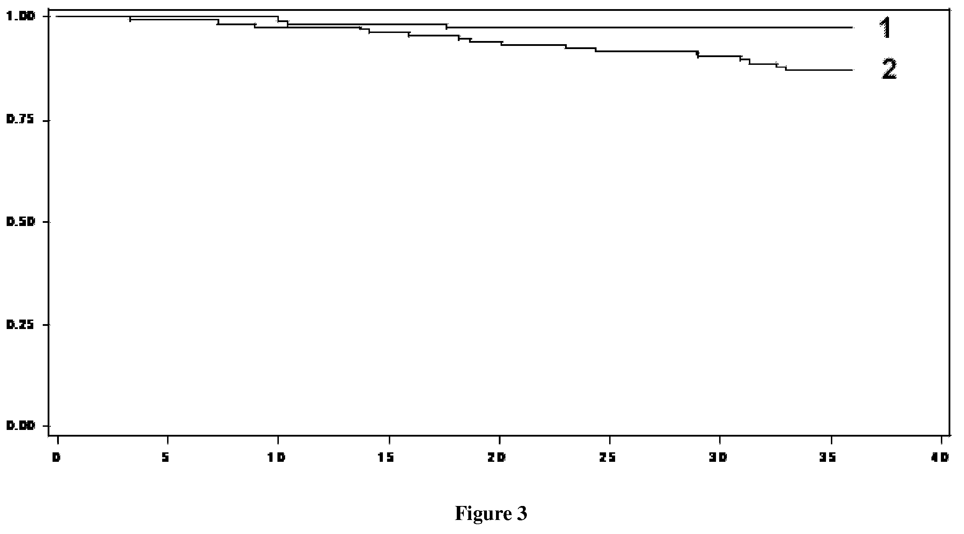

**Figure 3** is a diagram showing the Kaplan-Meier curves showing the survival time on a 36-months time period of HIV-1-infected patients having a CD4 count above 200 cells/mm³ and having a viral load level respectively below (upper curve n° 1) or above (lower curve n° 2) the predetermined reference value (here, the viral load reference value, or "cut-off" value, is of 4 log). Abscissa : time period following the date of HIV-1 infection, as expressed in months. Ordinate : survival distribution (100% =1 Unit).

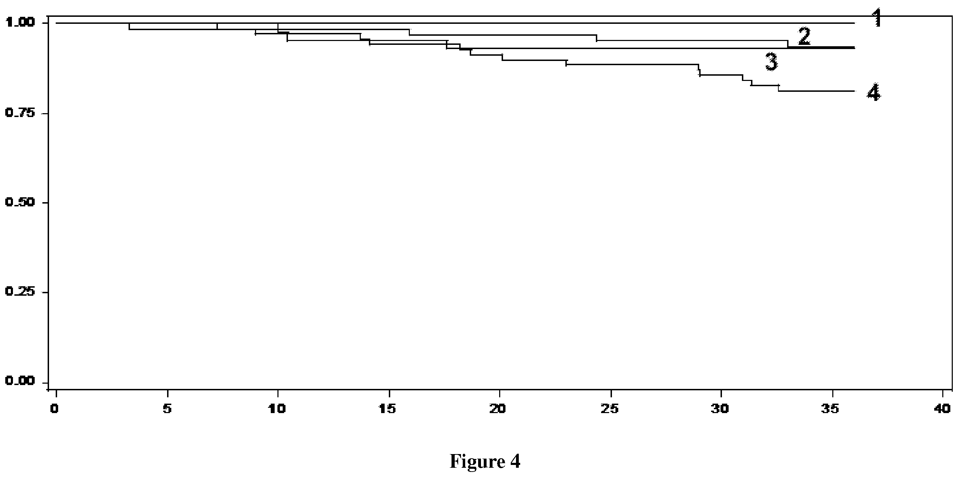

**Figure 4** illustrates a diagram of Kaplan-Meier curves showing the survival time on a 36-months time period of patients having a CD4 count above 200 cells/mm³ and classified in four classes, respectively (i) anti-3S antibodies above the predetermined reference value (>50) and viral load below or equal to the predetermined reference value (<=4 log) (curve n° 1), (ii) anti-3S antibodies above the predetermined reference value (>50) and viral load above the predetermined reference value (>4 log) (curve n° 2), (iii) anti-3S antibodies below or equal to the predetermined reference value (<=50) and viral load below or equal to the predetermined reference value (<=4 log) (curve n° 3), and (iv) anti-3S antibodies below or equal to the predetermined reference value (<=50) and viral load above the predetermined reference value (>4 log) (curve n° 4). Abscissa : time period following the date of HIV-1 infection, as expressed in months. Ordinate : ratio of living people (100%=1 Unit).

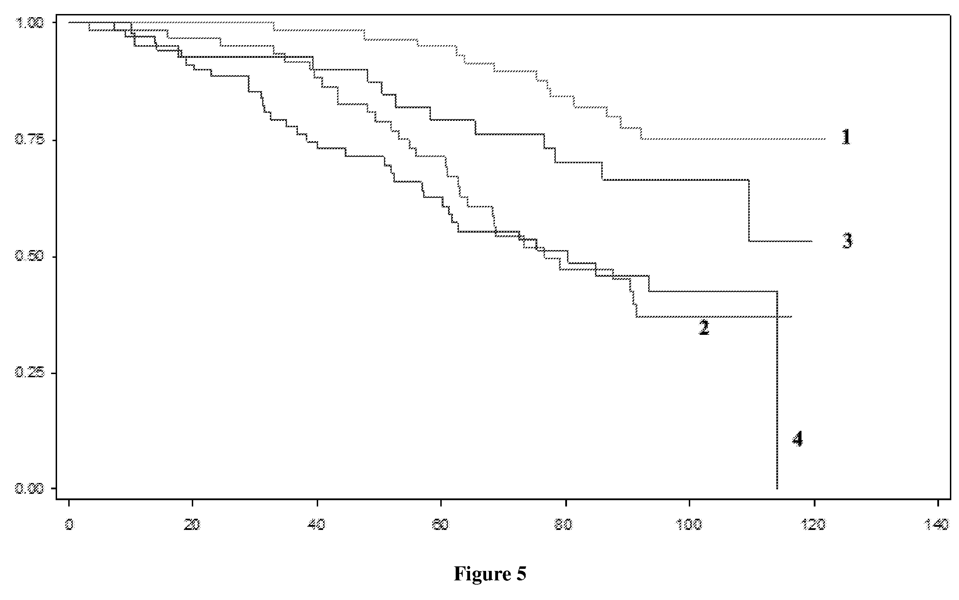

**Figure 5** illustrates a diagram of Kaplan-Meier curves showing the survival time on a 120-months time period of patients having a CD4 count above 200 cells/mm³ and classified in four classes, respectively (i) anti-3S antibodies above the predetermined reference value (>50) and viral load below or equal to the predetermined reference value (<=4 log) (curve n° 1), (ii) anti-3S antibodies above the predetermined reference value (>50) and viral load above the predetermined reference value (>4 log) (curve n° 2), (iii) anti-3S antibodies below or equal to the predetermined reference value (<=50) and viral load below or equal to the predetermined reference value (<=4 log) (curve n° 3), and (iv) anti-3S antibodies below or equal to the predetermined reference value (<=50) and viral load above the predetermined reference value (>4 log) (curve n° 4). Abscissa : time period following the date of HIV-1 infection, as expressed in months. Ordinate : ratio of living people (100%=1 Unit).

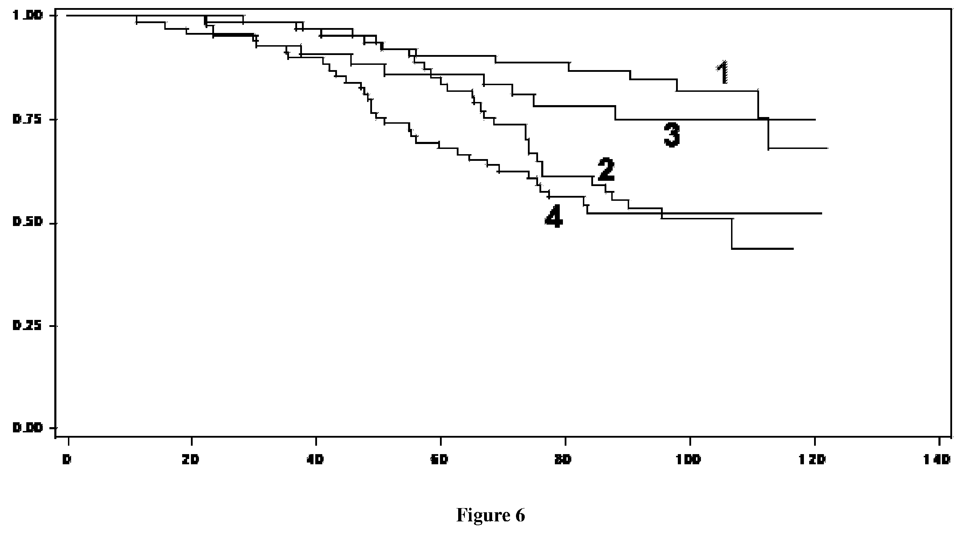

**Figure 6** illustrates a diagram of Kaplan-Meier curves showing the survival time on a 120-months time period of AIDS-free patients having a CD4 count above 200 cells/mm³ and classified in four classes, respectively (i) anti-3S antibodies above the predetermined reference value (>50) and viral load below or equal to the predetermined reference value (<=4 log) (curve n° 1), (ii) anti-3S antibodies above the predetermined reference value (>50) and viral load above the predetermined reference value (>4 log) (curve n° 2), (iii) anti-3S antibodies below or equal to the predetermined reference value (<=50) and viral load below or equal to the predetermined value (<=4 log) (curve n° 3), and (iv) anti-3S antibodies below or equal to the predetermined reference value (<=50) and viral load above the predetermined value (>4 log) (curve n° 4). Abscissa : time period following the date of HIV-1 infection, as expressed in months. Ordinate : ratio of living people (100%=1 Unit).

### DETAILED DESCRIPTION OF THE INVENTION

The present invention provides for a novel independent marker that is indicative of the progression status of an HIV-1 disease in HIV-1-infected patients. The present invention provides for novel methods for determining the prognosis of progression of an HIV-1 disease, using the said novel independent marker.

It has been found according to the present invention that the level of antibodies directed against a specific HIV-1-derived peptide (the "3S-peptide"), as determined in a sample from a tested HIV-1-infected individual, is indicative of the status of progression of the HIV-1 disease of the said individual.

The said 3S-peptide, which consists of a peptide that is derived from the gp41 glycoprotein from an HIV-1 virus, is known *per se.* The 3S peptide has been shown in the art to bind to gC1qR on CD4+ T cells and to induce the membrane expression of the NKp44L protein in CD4 T cells (Fauster Bovendo et al., 2010, PLOS Pathogens, Vol. 6(7); and WO 2010/040853). It was believed that the NKp44L expression at the surface of the CD4 T cells plays a key role in the lysis of CD4 T cells by activated NK cells during HIV-1-1 infection and consequently that specific antibodies directed against the 3S-peptide could affect disease course (see Vieillard et al., 2005, Proc. Natl. Acad. Sci. USA, Vol. 102 : 10981-10986).

Debré et al. (2009, Bull Acad Nat Med, Vol. 193(1) : 127-136) disclosed that anti-3S antibodies titre is highly correlated with the counts of CD4+ T cells and inversely correlated with the expression of NKp44L at the surface of CD4+ T cells.

It was also shown in the art that anti-3S antibodies were detected in some HIV-1-infected patients belonging to the very specific patients cohort named ALT, which cohort comprises exclusively HIV-1-infected patients having the rare phenotype of asymptomatic long-term survival (see Vieillard et al., 2006, AIDS, Vol. 20(14) : 1795-1804). Vieillard et al.(2006) have shown the existence, in the said ALT cohort of patients, of a correlation between (i) a decrease in the number of CD4⁺ T cells and (ii) a decrease in the anti-3S antibody level. Vieillard et al. (2006) have also shown that the presence of anti-3S antibodies was correlated with a high level of CD4⁺ T cells, in these highly specific HIV-infected individuals. The development of a prognostic test that would allow predicting the rate of progression towards AIDS in HIV-infected asymptomatic patients has also been contemplated in the art.

Vieillard et al.(2006) concluded that the presence of anti-3S antibodies might affect disease course in inhibiting NKp44L expression and CD4 sensitivity to NK lysis. Also,
Vieillard et al. (2006) stated that the presence of anti-3S antibodies in the serum helped to control the CD4 cell count. These authors observed that depletion of the overall CD4 T-cell population was closely related to the disappearance of anti-3S antibodies. These authors also added that the production of anti-3S antibodies could possibly contribute to slowing down the progressive depletion of CD4 T cells.

It has now been found according to the present invention that the level of anti-3S antibodies consists of a reliable marker of the progression status of the HIV-1 disease in a HIV-1-infected patient.

Surprisingly, the inventors have shown that the level of anti-3S antibodies is reliably indicative of the HIV-1 disease progression.

Further, it has been shown herein that the anti-3S antibody level consists of a marker indicative of the HIV-1 disease progression, even when the said marker is used alone.

Still further, it has been shown herein that the anti-3S antibody level consists of a HIV-1 disease progression marker that is independent from other conventionally used HIV-1 prognosis markers, including the CD4 count prognosis marker and the viral load prognosis marker.

It is herein specified that the results according to the present invention have been obtained from a general cohort of HIV1-infected patients, namely a cohort of HIV-1 seropositive individuals, and not from a cohort of patients having the rare phenotype of asymptomatic long-term survival like the ALT cohort studied by Vieillard et al. (2006).

By definition, the mechanisms of progression of an HIV infection in asymptomatic long-term survival individuals are distinct from those that may be found in the general population of HIV-infected subjects. This is again fully illustrated by the invention's findings disclosed herein.

It has been found according to the invention that, in a general population of HIV-1-infected patients, there is no correlation between (i) the level of anti-3S antibodies and (ii) the level of CD4⁺ T cells, in contrast to the findings of Vieillard et al. which had been obtained from the ALT cohort of patients. The invention's findings again illustrate that the one skilled in the art could not expect taking benefit of data obtained from asymptomatic long-term survival HIV-infected individuals for understanding the progression of the HIV infection in the general population of HIV-infected subjects.

Further, as indicated above, it was believed in the art, in the specific ALT cohort of patients, that the level of anti-3S antibodies was strongly correlated with the CD4 T cell count. It is recalled that the previous results of Vieillard et al. (2006) would have at most taught to the one skilled in the art that, because of the strong correlation that was found between the level of anti-3S antibodies and the CD4 cell count, there had been found a strong dependency between (i) a HIV-1 disease prognosis marker, namely the CD4 cell count and (ii) a biological parameter of HIV-1-infected patients, namely the level of anti-3S antibodies. Even upon hypothetical admission that the one skilled in the art would have considered the results of Vieillard et al. (2006), the previously disclosed redundancy of information that would have expectedly been brought by the CD4 cell count and the level of anti-3S antibodies would have dissuaded the one skilled in the art to explore the possibility that the level of anti-3S antibodies might consist of a prognosis marker of the HIV-1 disease progression.

In particular aspects, the invention provides a method for prognosis of the severity of the HIV-1 disease progression, which method may be used notably for monitoring the course of AIDS, as well as for monitoring the therapeutic efficiency of anti-retroviral treatments.

The present invention relates to an *in vitro* method for the prognosis of progression of an HIV-1 disease in a patient infected with an HIV-1 virus who has not the rare phenotype of asymptomatic long-term survival, which prognosis is based on the level of antibodies directed against 3S peptide of SEQ ID NO. 2 as a prognostic marker, which prognostic marker, the said method comprising the steps of:
a) measuring the level of antibodies directed against the 3S peptide of SEQ ID N° 2 in a sample collected from the said patient,
b) comparing the level of anti-3S antibodies measured at step a) with a reference value of anti-3S antibody level that is indicative of the progression of the HIV-1 disease.

As it is shown in the examples herein, a correlation has been found between the level of anti-3S antibodies, and not the presence thereof, and a prognosis of progression of an HIV-1 disease, in the general population of HIV-1-infected individuals.

As intended herein, a "HIV-1 disease" encompasses basically all the physiological conditions that are undergone by an individual who has been infected by a HIV-1 virus, starting from the time of the virus infection event until the date of the individual's death, irrespective of whether the individual's death is a direct or indirect consequence of the virus infection event. It is recalled that the infection of an individual with a HIV-1 virus causes a chronic disease state that progressively causes a reduction of the effectiveness of the immune system and leaves the HIV-1-infected individuals susceptible to opportunistic infections and tumors. Thus, a HIV-1 disease encompasses the primary infection (or acute infection) time period, the seroconversion time period, the asymptomatic stage time period, the early- and medium-stage of HIV-1 symptomatic disease, as well as the late stage of HIV-1 disease (also called AIDS).

According to the invention, anti-3S antibodies that are directed against the peptide of SEQ ID N° 2 encompass antibodies that bind specifically to a reference polypeptide having an amino acid sequence comprising the peptide sequence NH₂-SWSNKS-COOH (SEQ ID N° 1). These antibodies are collectively termed "anti-3S antibodies" in the present description. It may also corresponds to antibodies binding to a peptide having an amino acid sequence that is closely related to sequence of SEQ ID N° 2. Peptides having a sequence closely related to SEQ ID N° 2 encompass peptides comprising an amino acid sequence selected from the group consisting of SEQ ID N° 3 and SEQ ID N°4, which includes peptides having 16 amino acid residues in length and comprising an amino acid sequence selected from the group consisting of SEQ ID N° 3 and SEQ ID N°4, e.g. which includes (i) a variant of the peptide of SEQ ID N°2 wherein the amino acid residue Serine at position 11 is replaced by a threonine residue and (ii) a variant of the peptide of SEQ ID N°2 wherein the amino acid residue Lysine at position 10 is replaced by an arginine residue.

As intended herein, antibodies directed against a 3S-peptide encompass antibodies that bind to the peptide of SEQ ID N° 2.

According to the present invention, the anti-3S antibodies preferably consists of polyclonal antibodies that bind to a 3S peptide, and which are contained in a sample collected from a HIV-infected individual.

The 3S-peptide consists of the peptide consisting of the amino acid sequence of SEQ ID N° 2.

As intended herein, the "level" of a HIV disease prognosis marker consists of a quantitative value of the said prognosis marker in a sample, e.g. in a sample collected from an HIV-infected patient. In some embodiments, the said quantitative value does not consist of an absolute value that is actually measured, but rather consists of a final value resulting from the taking into consideration of a signal to noise ratio occurring with the assay format used, and/or the taking into consideration of calibration reference values that are used to increase reproducibility of the measures of the level of a HIV disease marker, from assay-to-assay. In some embodiments, the "level" of a HIV disease prognosis marker is expressed as arbitrary units, since what is important is that the same kind of arbitrary units are compared (i) from assay-to-assay, or (ii) from one HIV-infected patient to others, or (iii) from assays performed at distinct time periods for the same patient, or (iv) between the HIV prognosis marker level measured in a patient's sample and a predetermined reference value (which may also be termed a "cut-off" value herein).

Indeed, whatever the kind of numerical units are used for expressing the level of anti-3S antibodies in a sample, the measured value reflects the amount of anti-3S antibodies that is contained in the tested sample, or most preferably the concentration of anti-3S antibodies in the said sample.

Also illustratively, the HIV viral load prognosis marker may be expressed in numerical units that reflect the number of copies of HIV genome contained in the patient's sample tested, or alternatively the concentration of HIV genome copies in the tested patient's sample.

Similarly, other HIV prognosis markers may be expressed in numerical units which reflect, respectively, (i) the number of CD4 T cells in the sample tested or alternatively the concentration thereof, and (ii) the percentage of CD4 T cells among the total lymphocyte cells present in the tested sample.

As intended herein, a sample collected from a patient encompasses any body fluid expected to contain antibodies and primarily in blood and bolld-derived substances. According to the invention, a sample collected from a patient encompasses a whole blood sample, a serum sample and a blood plasma sample.

As shown in the examples herein, the *in vitro* method of the invention allows determining the likelyhood of an early or a delayed occurrence of an immunosuppression state in HIV-infected patients. Consequently, the *in vitro* method of prognosis of progression of an HIV disease of the invention may be practiced with the view of determining the period of time intervals for following up the clinical parameters of an HIV-infected patient and then for deciding of the opportunity to start an anti-retroviral therapeutic treatment.

As it is also shown in the examples herein, the *in vitro* method of the invention allows determining the prognosis of progression of the HIV disease in HIV-infected patients that are already undergoing an immunosuppression state due to the viral infection. In other words, the *in vitro* method of the invention allows discriminating between (i) HIV-infected immunosuppressed patients having a high likelihood to enter early into a symptomatic stage of the HIV disease and (ii) HIV-infected immunosuppressed patients having a low likelihood to enter early into a symptomatic stage of the HIV disease.

Also, the examples herein show that the *in vitro* method for prognosis of progression of the HIV disease of the invention allows to determine between (i) HIV-infected patients having a high likelihood of a long term survival and (ii) HIV-infected patients having a low likelihood of a long term survival.

At step a) of the method, the level of anti-3S antibodies may be measured by any antibody detection method that is well known from the one skilled in the art. Thus, the level of anti-3S antibodies may be measured by any kind of immunoassays including, but not limited to, ELISA, radioimmunoassays, fluorescence immunoassays, immunoaffinity chromatography, immuno-precipitation and the like.

In certain embodiments, the level of anti-3S antibodies is measured by an immunosorbent assay, which encompasses an ELISA method.

At step a), the level of the anti-3S antibodies is expressed as a value that refers to an actual signal that is generated, directly or indirectly, by the complexes formed between (i) 3S peptides used as baits in the antibody detection method and (ii) the anti-3S antibodies present in the patient's sample tested.

As is conventional when performing antibody detection methods, e.g. immunoassays like ELISA, the signal level (i.e. the anti-3S antibodies level) is expressed as Arbitrary Units (AU). Arbitrary Units are generally determined after substracting the background noise signal, the latter being measured from a sample of the same kind than that of the patient's sample (e.g. a blood serum sample or a blood plasma sample) that is known to be exempt from anti-3S antibodies. Generally, the Arbitrary Units are determined after calibration of the antibody detection method, using a serial of calibration standards (e.g. a serial of samples wherein each sample contains a known amount of anti-3S antibodies).

At step b) of the *in vitro* HIV disease prognosis method of the invention, the quantification value of the level of anti-3S antibodies that is measured at step a) is compared to a predetermined reference value that is indicative of an HIV disease prognosis. The said predetermined reference value is "indicative of an HIV disease prognosis" since it allows discrimination between (i) a "good" prognosis of HIV disease progression for values measured at step a) that are above the said reference value and (ii) a "bad" prognosis of HIV disease progression for values measured at step a) that are below the said reference value.

As it is disclosed in the examples herein, by performing Kaplan-Meier curves from anti-3S antibodies level values obtained in samples collected from a large cohort of HIV-infected individuals who have been followed up for various relevant clinical parameters including survival time, the inventors have determined an anti-3S antibody level value that allows to discriminate between (i) patients with a favourable outcome, including a delayed spontaneous HIV disease progression and a long survival time and (ii) patients with a poor outcome, including a non-delayed spontaneous HIV disease progression and a short survival time.

Illustratively, when using the ELISA test format disclosed in the examples herein, the predetermined reference value, which may also be termed the "cut-off" value, is of 50. Thus, using the ELISA test format disclosed in the examples, a sample collected from an HIV-infected patient where an anti-3S antibody level value of less than 50 has been measured at step a) of the *in vitro* method according to the invention is classified as a patient with a non-favourable outcome. Conversely, a sample collected from an HIV-infected patient where an anti-3S antibody level value of more than 50 has been measured at step a) of the *in vitro* method according to the invention is classified as a patient with a favourable outcome.

For performing the *in* vitro HIV disease prognosis method of the invention_, the reference value used for comparison at step b) of the method , which may also be termed a "cut-off" value), may be determined as described hereunder.

The reference ("cut-off") value for the anti-3S antibodies prognosis marker may be predetermined by carrying out a method comprising the steps of:
a) providing a collection of samples from HIV-infected patients;
b) providing, for each patient's sample provided at step a), information relating to the actual clinical outcome for the corresponding HIV-infected patient;
c) providing a serial of arbitrary quantification values for the level of anti-3S antibodies;
d) quantifying the level of anti-3S antibodies in each patient's sample contained in the collection provided at step a);
e) classifying the said patient's samples in two groups for one specific arbitrary quantification value provided at step b), respectively :
   (i) a first group comprising patient's samples that exhibit a quantification value for the level of anti-3S antibodies that is lower than the said arbitrary quantification value contained in the said serial of quantification values;
   (ii) a second group comprising patient's samples that exhibit a quantification value for the level of anti-3S antibodies that is higher than the said arbitrary quantification value contained in the said serial of quantification values;
   whereby two groups of patient's samples are obtained for the said specific quantification value, wherein the patient's samples of each group are separately enumerated;
f) calculating the statistical significance between (i) the quantification value for the said biological marker obtained at step d) and (ii) the actual clinical outcome of the patients from which serum/plasma samples contained in the first and second groups defined at step e) derive;
g) testing through steps e) and f) various arbitrary values,
h) setting the said reference value ("cut-off" value) as consisting to the arbitrary quantification value obtained for which the highest statistical significance (most significant) has been observed at step g).

As it is disclosed above, the said method allows the setting of a "cut-off" value permitting discrimination between bad and good outcome prognosis.

As intended in the present specification, and as shown in the examples herein, (i) a bad outcome prognosis encompasses a high likelihood that the onset of clinical symptoms of AIDS occurs within a 6-72 months time period following the time of infection with HIV, as well as a short time period of survival for patients having a CD4 T cell count below 500 cells/mm³ (which includes patients having a CD4 T cell count below 200 cells/mm³, whereas (ii) a good outcome prognosis encompasses a low likelihood that the onset of clinical symptoms of AIDS occurs within a 6-72 months time period following the time of infection with HIV, as well as a long time period of survival for patients having a CD4 T cell count below 500 cells/mm³ (which includes patients having a CD4 T cell count below 200 cells/mm³.

In certain preferred embodiments of step b) of the method for determining cut-off values above, the said information relating to the actual clinical outcome of the HIV-infected patients are selected from the group consisting of (i) the duration of the AIDS-free survival (AFS) and (ii) survival time of patients having a CD4 T cell count below 500 CD4 cells/mm³ or below 200 CD4 cells/mm³ (TCDB).

As it is shown in the examples herein, the accuracy of the HIV disease prognosis, when using the level of anti-3S antibodies as a prognosis marker, may be further improved by combining the said prognosis marker with one or more HIV prognosis markers known *perse,* e.g. the HIV viral load.

As shown in the examples, the HIV disease prognosis accuracy of the level of anti-3S antibodies is significantly higher than the prognosis accuracy of one of the other known HIV prognosis markers, namely the viral load. However, the examples also show that the prognosis accuracy of the *in vitro* HIV disease prognosis method of the invention may be improved by combining (i) the viral load marker and (ii) the level of anti-3S antibodies marker.

Thus, in some embodiments of the *in vitro* method for HIV disease prognosis of the invention, the said method further comprises the steps of:
1) measuring the HIV viral load in the said sample collected from the said patient, and
2) comparing the viral load value measured at step 1) with a reference value that is indicative of the progression of the HIV disease.

In the method embodiment above, the viral load may be measured through any one of the conventional techniques that are well known from the one skilled in the art. Illustratively, the viral load may be measured by using the method disclosed by Jennings et al. (2005, J Clin Microbiol, Vol. 43(12) : 4950-4956). Indeed, the reference value for the viral load quantification may be determined by using the same methods as those described herein for calculating the predetermined reference value (or "cut-off" value) of the level of anti-3S antibodies.

As it can be easily understood by the one skilled in the art, the predetermined reference value (or "cut-off" value) for the viral load marker may vary, depending of the viral load assay format which is used.

Illustratively, a predetermined reference value ("cut-off" value) of 4 log for the viral load marker has been used in the examples for performing the *in vitro* HIV disease prognosis method of the invention.

Without wishing to be bound by any particular theory, the inventors believe that the *in vitro* HIV disease prognosis method of the invention may be performed by combining the anti-3S antibodies prognosis marker to one or more other HIV disease prognosis marker. The said one or more HIV disease prognosis markers may be selected from the group consisting of (i) the HIV viral load, (ii) the absolute CD4 T cells count and (iii) the percentage of CD4+ T lymphocytes.

According to these embodiments, the *in vitro* method for the prognosis of progression of a HIV disease may comprise the steps of:
a) measuring the level of anti-3S antibodies and at least one other HIV disease prognosis marker in a sample collected from a HIV-infected patient, and
b) comparing, for each HIV disease prognosis marker measured at step a), the resulting marker value with a reference value for the said prognosis marker.

In preferred embodiments, the said one or more other HIV disease prognosis markers are selected from the group consisting of (i) the HIV viral load, (ii) the absolute count of CD4 T cells, (iii) the percentage of CD4 T cells and (iv) the ratio of CD4+/CD8+ T lymphocytes. These include the following combinations of HIV disease prognosis markers:
- anti-3S antibodies level combined with marker (i) above,
- anti-3S antibodies level combined with marker (ii) above,
- anti-3S antibodies level combined with marker (iii) above,
- anti-3S antibodies level combined with marker (iv) above
- anti-3S antibodies level combined with markers (i) and (ii) above,
- anti-3S antibodies level combined with markers (i) and (iii) above,
- anti-3S antibodies level combined with markers (i) and (iv) above,
- anti-3S antibodies level combined with markers (ii) and (iii) above,
- anti-3S antibodies level combined with markers (ii) and (iv) above,
- anti-3S antibodies level combined with markers (iii) and (iv) above,
- anti-3S antibodies level combined with markers (i), (ii) and (iii) above,
- anti-3S antibodies level combined with markers (i), (ii) and (iv) above,
- anti-3S antibodies level combined with markers (ii), (iii) and (iv) above,
- anti-3S antibodies level combined with markers (i), (iii) and (iv) above, and
- anti-3S antibodies level combined with markers (i), (ii), (iii) and (iv) above,

Depending on whether each of the marker values measured at step a) are above or below the respective reference values to which they are compared at step b), a "good" prognosis of progression of the HIV disease or a "bad" prognosis of progression of the HIV disease is determined. Indeed, (i) a anti-3S antibody value above the predetermined value is classified as a "good" prognosis, whereas (ii) a viral load value above the predetermined reference value is classified as a "bad" prognosis parameter.

Indeed, the results of step b) encompasses situations wherein (i) for at least one HIV prognosis marker tested, the marker value measured at step a) is classified as a "bad" prognosis for the said marker (i.e. the marker may be marked "Low") and (ii) for at least one other HIV prognosis marker tested, the marker value measured at step a) is classified as a "good" prognosis for the said marker (i.e. the marker may be marked "High"). In these situations, the prognosis for progression of the HIV disease is determined by taking into account the number, and optionally the respective statistical weights, of "High" markers and of "Low" markers. In some embodiments, the measured value for each marker tested is balanced by affecting to the said measured marker value a numerical factor that materializes its weight of statistical significance in the prognosis of progression of the HIV disease.

In the embodiments of the method for the prognosis of progression of the HIV disease of the invention wherein the anti-3S antibody prognosis marker is combined with one or more other HIV disease prognosis markers, step b) is performed by mathematically integrating the values measured at step a) for each HIV prognosis marker tested and their comparison to each corresponding reference values, so as to generate a single prediction value (e.g. "bad" prognosis or "good" prognosis) of the resulting composite HIV disease prognosis marker. The generation of composite markers that integrate a combination of two or more markers is well known from the one skilled in the art.

Illustratively, a composite prognosis marker for HIV disease progression may result from a linear combination of each of the individual prognosis markers included therein, where logistic regression may be used to estimate the respective weight of each individual prognosis marker within the composite prognosis marker. Alternatively, an appropriate mathematical model could be used to establish the prognostic value of the used combination of markers.

The present invention further provides a method for monitoring the efficacy of a therapeutic treatment for HIV infection, by providing an accurate prognostic indicator of disease progression. Such a monitoring method is very important both (i) for identifying possible therapeutic drugs and (ii) for monitoring patients undergoing therapies with these drugs.

Notably, the present invention deals with a method for monitoring the efficacy of a therapeutic treatment comprising the steps of:
a) performing a therapeutic treatment by administering, to an HIV-infected patient in need thereof, a pharmaceutical composition comprising one or more anti-retroviral agents, and
b) performing the *in vitro* method for prognosis of progression of a HIV disease described herein on a sample collected from the said patient, and
c) suitably adapt the therapeutic treatment performed at step a), if necessary.

In some embodiments, the therapeutic treatment that is performed at step a) is continued if a "good" prognosis is determined, e.g. a high likelihood of a long survival time without entering into the AIDS stage.

In some embodiments, the therapeutic treatment is adapted (e.g. is administered at a higher dosage or one or more of the active ingredients are replaced by more efficient ones) if a "bad" prognosis is determined, e.g. a low likelihood of a long survival time without entering into the AIDS stage.

In a specific aspect, the invention contemplates administration of an effective amount of an antiviral agent (or of a combination of antiviral agents) to an HIV-infected individual and monitoring the therapeutic outcome.

As it has been already specified previously in the present description, the in vitro method of prognosis for progression of a HIV disease is preferably performed by known immunodetection methods. The examples herein illustrate embodiments of performing the in vitro HIV prognosis method of the invention by an enzyme-linked immunosorbent assay (ELISA), that is a highly conventional immunodetection method. Like other immunodetection methods, an ELISA may be performed either as a non-competitive immunoassay or as a competitive immunoassay.

In a non-competitive ELISA, unlabeled antigen (e.g. a 3S peptide) is bound to a solid support or reaction vessel, such as the surface of a microtiter plate or biochip.

Then, the biological sample (e.g. the sample collected from an HIV-infected patient) is combined with the antigen bound to the reaction vessel, and the antibodies present in the biological sample (e.g. the anti-3S antibodies present in the sample collected from an HIV-infected patient) are allowed to bind to the antigens immobilised on the solid support, thus forming immune complexes.

After the immune complexes have formed, excess biological sample is removed and the vessel is washed to remove non-specifically bound antibodies. The immune complexes are then reacted with an appropriate enzyme-labeled anti-immunoglobulin (which may be also termed "secondary antibody"). The secondary antibody reacts with the antibodies (e.g. the anti-3S antibodies) in the immune complexes, not with other antigens bound to the reaction vessel. Secondary antibodies specific for binding human antibodies are well known in the art and are commercially available, such as from Sigma Chemical Co. (St Louis, MO, USA).

After a second wash step, the enzyme substrate is added. The enzyme linked to the secondary antibody catalyzes a reaction that converts the substrate into a detectable product. When excess antigen is present, the amount of product is directly proportional to the amount of antibodies (also termed "primary antibodies") present in the patient's sample.

In some embodiments, the product is fluorescent or luminescent, which can be measured using technology and equipment well known in the art. In some embodiments, the enzyme reaction results in the conversion of the enzyme substrate into a coloured product, which can be measures spectrophotometrically.

Typical enzymes that can be linked to secondary antibodies include horseradish peroxidise, glucose oxidase, glucose-6-phosphate dehydrogenase, alkaline phosphatise, beta-galactosidase and urease.

For performing ELISA, suitable solid supports include organic and inorganic polymers, e.g. dextrans, natural or modified celluloses, polyethylene, polystyrene, polyacrylamides, etc.

For performing the *in vitro* prognosis for progression of the HIV disease according to the invention, by immunodetection of the anti-3S antibodies using a 3S peptide of SEQ ID N° 2, the said 3S peptide may be obtained either (i) by chemical synthesis or (ii) by recombinant expression, e.g. in *E. coli* cells transformed with an expression cassette (eventually inserted in a recombinant vector, i.e. a recombinant plasmid) encoding the said 3S peptide.

The present invention is further illustrated by, without being in any way limited to, the examples hereafter.

### EXAMPLES

### Example 1 : Identification of the level of anti-3S antibodies as a prognosis marker of the progression of a HIV disease

### A. Materials and Methods

### A.1. Patients

The ANRS SEROCO/HEMOCO cohort started in 1988 has enrolled HIV-infected adults referred from 21 hospitals and a network of private practitioners. The study was approved by the Ethics Committee and all subjects gave written informed consent. Patients receive thorough clinical and laboratory examinations at inclusion and are then seen every 3 or 6 months according to their clinical status. All AIDS defining illnesses are checked in the medical files and reviewed with the participating physicians. The clinical status of patients lost to follow-up is assessed by crosschecking with the national AIDS register. Sera collected at enrolment and at each 6 month visit are stored at minus 180°C.

In this study eligible patients had a known date of seroconversion, were included in the cohort within 24 months after infection, and before 1996 and had available cryopreserved serum samples from enrolment. HIV seroconversion was documented by an interval of less than 24 months between a negative and a positive HIV antibody test, or an incomplete Western blot followed by a complete Western blot. The date of infection was defined as the date of the incomplete Western blot minus 1 month, or the date of a primary symptomatic infection minus 15 days, or the midpoint between the two tests (Hubert 2000). 244 seroconverters with available frozen sera were included in this analysis. Follow-up was censored in 1996, before cART widespread use era. The median year of infection was 1989 and the median follow-up time since infection was 6.5 years.

### A.2. Study protocol

We considered 244 HIV-1 untreated seroconverters enrolled before 1996 in the SEROCO cohort, within 24 months following infection (median year of enrolment 1989). Follow-up was censored in 1996, median follow-up was 6.5 years. Early anti-3S antibodies levels were measured at enrolment from frozen sera. To estimate the role of anti-3S antibodies on the risk of disease progression, time since infection to CD4 below 200/mm³ (91 events) and clinical AIDS (83 events) were estimated through Kaplan-Meier curves, comparing patients with antibodies > 50 units/mL to those with lower or undetectable levels. Crude and adjusted RR were estimated through Cox regression models.

### A.3. Measure of the level of anti-3S antibodies

The ELISA assay was performed on flat-bottom 96-well microplates (Maxisorp, Nunc).

The plates were coated with the 3S-peptide at 2 mg/ml in PBS, overnight 17 h at 4°C. Plates were washed twice in PBS/0,1%Tween, and then blocked in PBS/3% nonfat milk for 2 h at 37°C.

The tested serum/plasmas were heat-inactivated (56°C, 45 min) and then added at various concentrations (1/20, 1/200 and 1/2000) in triplicate for 1h30 min h at 37°C. Serums are diluted in assay buffer (PBS, 3% non fat milk, 0,1% tween-20).

After washing twice in PBS/0,1%Tween, 1/2000 diluted biotin-SP-conjugated rabbit anti-human IgG (Jackson ImmunoResearch) was added and incubated for 1 h at 37°C. The biotin-conjugated antibody is diluted in assay buffer (PBS, 3% not fat milk, 0,1% tween-20)

After washing, biotin was captured using a 1/5000 diluted ExtraAvidin Peroxidase conjugate (Sigma) for 1 h at 37°C. ExtrAvidin is diluted in assay buffer (PBS, 3% non fat milk, 0,1% tween-20) After the last wash, color was developed with substrate solution, and the optical density (OD) at 450 nm was measured.

The plates were washed twice in PBS/0,1%Tween and then 2-times in PBS, and color is revealed in the presence of TMB (Sigma,) at room temperature in the dark for 30 min.

The chemical reaction is stopped with 4N H₂SO₄, and analyzed at 450 nm (Microplate reader, Molecular Devices).

Negative controls used human AB serum (SAB) and plasma from 10 uninfected donors diluted at 1/20 in assay buffer.

Quantification of anti-3S antibodies used calibration standards including dilutions of a purified mouse anti-3S monoclonal antibody, named 15C8f2. The standard curve corresponds to serial 2-fold dilutions of this monoclonal antibody, with value comprised between 0 and 200 ng/ml. The monoclonal antibody is diluted in assay buffer.

The OD of the tested samples are reported within the linear part of the standard curve to obtain the corresponding concentration of 15C8F2 monoclonal antibody giving the same signal.

The values of anti-3S antibodies, expressed in arbitrary unit, are determined by taking into account the dilution factor of the tested samples and the volume. The detection limit is set by the values obtained for the negative control and fixed to a value of 10 arbitrary units.

### A.4. Other methods

CD4 lymphocyte counts are determined at each visit by means of flow cytometry. All HIV-1 RNA levels were determined retrospectively from stored sera.

The three participating university labs used reverse transcriptase-polymerase chain reaction (Amplicor HIV-1 Monitor assay, Roche Molecular Systems, Neuilly-sur-Seine, quantification threshold 400 copies/mL). Early anti-3S antibodies levels were measured at enrolment from frozen sera (median time since estimated date of infection: 9 months).

### A.5 Statistical analysis

Pearson's chi-square or Fisher's exact test were used to compare qualitative variables; non parametric Kruskal Wallis test was used for continuous variables.

To assay for qualification of anti-3S antibody level as a prognosis marker of the risk of HIV disease progression, time since infection to CD4 below 200/mm³ (91 events) and to clinical AIDS (83 events) was estimated through Kaplan-Meier curves. Event-free survival curves in patients with antibodies > 50 units/mL were compared to those with lower or undetectable levels using the log rank test. Crude and adjusted relative risks (RR) were estimated by using a Cox model.

### B. Results

72% of seroconverters had detectable anti-3S antibodies close to seroconversion (median time since estimated date of infection: 9 months), 53% were above 50 units. Anti-3S antibodies levels at enrolment were inversely correlated with HIV-RNA levels, while no association was found with CD4 counts, as it is shown in Table 1 below. Thus, the results show that the level of anti-3S antibodies and the CD4 T cells count consists of independent markers.

**Table 1**

| Anti-3S level | **<10** | **11-50** | **51-85** | **86-120** | **121-180** | **>180** | **P** |
|---|---|---|---|---|---|---|---|
| | N=69 | N=45 | N=48 | N=39 | N=32 | N=11 | |
| CD4 inclusion* | 530 | 534 | 563 | 520 | 609 | 610 | 0.61 |

| | | | | | | | |
|---|---|---|---|---|---|---|---|
| * Median | | | | | | | |

Progression to CD4 below 200/mm³ was significantly delayed in patients with anti-3S Ab >50, but the effect was transient (interaction term between anti-3S Ab and time: p=0.003). These results are illustrated in figures 1 and 2.

The results of figures 1 and 2 show that the risk of progression towards a CD4 T cell count below 200 CD4 T cells/mm³ is delayed in patients having a level of anti-3S antibodies above the cut-off value of 50, when taking into account the first 120 months after inclusion (figure 1; P logrank=0.07; P Wilcoxon=0.02) or the first 36 months after inclusion (figure 2; P logrank=0.002; P Wilcoxon=0.02). The results of figures 1 and 2 show that the level of anti-3S antibodies consists of an actual prognosis marker for the progression of a HIV disease, and in particular for prognosis of survival in HIV-infected patients having a CD4 T cell count above 200/mm³.

During the first 3 years following infection the relative risk (RR) for CD4<200/mm³ was 0.21 ([95% CI: 0.07-0.62], p=0.005).

This survival advantage was still observed after adjustment for baseline viral load and CD4, age and sex (adjusted RR: 0.23 [0.07-0.73], p=0.01). Similar results were observed for time to AIDS.

Kaplan-Meier curves show that the survival benefit in patients with antibodies >50 units/mL and viral load >4log compared with other patients with viral load > 4 log was not sustained any longer at 5-6 years. These results are illustrated in figures 4 to 6. Figure 3 illustrates the HIV prognosis value of the HIV viral load marker alone (p logrank=0.005; P Wilcoxon=0.006). Both biomarkers have a different intrinsic prognostic value, each of these being independent.

Figure 3 illustrates an analysis of the level of HIV viral load for the first 36 months after enrolment, the viral load values being classified as above or below the cut-off value of 4 log.

Figure 4 illustrates the combined analysis of (i) the level of anti-3S antibodies (above ("Hi") or below ("Lo") the cut-off value of 50) and (ii) the level of the HIV viral load (above or below the cut-off value of 4 log), for 36 months subsequent to contamination. The results of figure 4 show that a more accurate prognosis of progression of the HIV disease is determined when the anti-3S antibody level marker (HIV-infected patients having an anti-3S antibody level above the cut-off value of 50) is combined with the HIV viral load marker (HIV viral load value below the cut-off value of 4 log) (p logrank=0.001; P Wilcoxon=0.001). The same results for 120 months subsequent to contamination are shown in figure 5 (p logrank=0.0001; p Wilcoxon=0.0001).

Figure 6 illustrates prognosis of the risk of progression towards AIDS when using the combined measure of (i) the level of anti-3S antibodies and (ii) the level of the HIV viral load), for 120 months subsequent to contamination. The results of Figure 6 show the highly accurate prognosis of progression of the HIV disease, with highly significant values of P logrank=0.0005 and P Wilcoxon=0.0003, respectively.

A subset of subjects had their antibodies measured again at 24-36 months following enrolment.

Subjects with persistently high antibodies still had a disease-free survival advantage compared with subjects who experienced a loss in anti-3S antibodies levels below 50.

Finally, it is shown in Table 2 hereunder that the level of anti-3S antibodies and the HIV viral load consist of two independent prognosis markers for the progression of the HIV disease in HIV-infected patients.

**Table 2**

| **Anti-3S Antibodies** | **RR crude (CI 95%)** | **p** | **RR adjusted** (CI 95%)** | **p** |
|---|---|---|---|---|
| Anti-3S Ab <50 | 1 | | 1 | |
| Anti-3S Ab >50 | 0.21 (0.07-0.62) | 0.005 | 0.31 (0.10-0.98) | 0.046 |

| | | | | |
|---|---|---|---|---|
| ** adjusted on age, sex, viral load and VIH DNA | | | | |

The results of Table 2 also show that the risk of progression, over 36 month period, towards a CD4 T cell count of less than 200 CD4 T cells/mm³ is delayed in patients with a level of anti-3S antibodies above the cut-off value of 50, after adjustment with age, sex, VIH RNA and DNA.

All these results show that CD4 level, viral load and anti-3S are three independent markers of prognosis of HIV infection. Moreover a threshold value of anti-3S has been determined as a cut-off value between poor and good prognostic patients.

## Claims

1. An *in vitro* method for the prognosis of progression of an HIV-1 disease in a patient infected with an HIV-1 virus who has not the rare phenotype of asymptomatic long-term survival, which prognosis is based on the level of antibodies directed against 3S peptide of SEQ ID NO. 2 as a prognostic marker, which prognostic marker is independent from the CD4 count prognosis marker, the said method comprising the steps of:
a) measuring the level of antibodies directed against the 3S peptide of SEQ ID N° 2 in a sample collected from the said patient,
b) comparing the level of anti-3S antibodies measured at step a) with a reference value of anti-3S antibody level that is indicative of the progression of the HIV-1 disease.

2. The method according to claim 1, wherein step a) is performed by immunodetection of the antibodies directed against the peptide of SEQ ID N° 2.

3. The method according to claim 1, wherein step a) is performed by an ELISA assay using a polypeptide comprising the peptide of SEQ ID N° 2 immobilised on a solid support.

4. The method according to claim 1, further defined as comprising the following steps:
1) measuring the HIV-1 viral load in the said sample collected from the said patient, and
2) comparing the viral load value measured at step 1) with a reference value that is indicative of the progression of the HIV-1 disease.

5. The *in vitro* method for the prognosis of progression of a HIV-1 disease according to claim 1, wherein :
- step a) further comprises measuring at least one other HIV-1 disease prognosis marker in a sample collected from a HIV-1-infected patient, and
- step b) comprises comparing, for each HIV-1 disease prognosis marker measured at step a), the resulting marker value with a reference value for the said prognosis marker.

6. The *in vitro* method according to claim 5, wherein the said one or more other HIV-1 disease prognosis markers are selected from the group consisting of (i) the HIV viral load, (ii) the absolute count of CD4 T cells, (iii) the percentage of CD4 T cells and (iv) the ratio of CD4+/CD8+ T lymphocytes.

7. The *in vitro* method according to claim 6, wherein step a) comprises the measure of a combination of HIV-1 disease prognosis markers selected from the group consisting of:
- anti-3S antibodies level combined with marker (i) above,
- anti-3S antibodies level combined with marker (ii) above,
- anti-3S antibodies level combined with marker (iii) above,
- anti-3S antibodies level combined with marker (iv) above
- anti-3S antibodies level combined with markers (i) and (ii) above,
- anti-3S antibodies level combined with markers (i) and (iii) above,
- anti-3S antibodies level combined with markers (i) and (iv) above,
- anti-3S antibodies level combined with markers (ii) and (iii) above,
- anti-3S antibodies level combined with markers (ii) and (iv) above,
- anti-3S antibodies level combined with markers (iii) and (iv) above,
- anti-3S antibodies level combined with markers (i), (ii) and (iii) above,
- anti-3S antibodies level combined with markers (i), (ii) and (iv) above,
- anti-3S antibodies level combined with markers (ii), (iii) and (iv) above,
- anti-3S antibodies level combined with markers (i), (iii) and (iv) above, and
- anti-3S antibodies level combined with markers (i), (ii), (iii) and (iv) above,

8. A method for monitoring the efficacy of a therapeutic treatment comprising the step of performing the *in vitro* method for prognosis of progression of a HIV-1 disease according to any one of claims 1 to 7 on a sample collected from an HIV-1-infected patient who has not the rare phenotype of asymptomatic long-term survival, and who is undergoing a therapeutic treatment with a pharmaceutical composition comprising one or more anti-retroviral agents.

## Patentansprüche

1. In-vitro-Verfahren zur Prognose des Fortschritts einer HIV-1-Erkrankung in einem mit einem HIV-1-Virus infizierten Patienten, welcher nicht den seltenen Phänotyp eines asymptomatischen Langzeitüberlebens hat, wobei die Prognose auf dem Niveau der Antikörper gegen 3S-Peptide einer SEQ ID Nr. 2 als prognostischer Marker basiert, wobei der prognostische Marker unabhängig von dem Vorhersage-Marker einer CD4-Zählung ist, wobei das Verfahren die folgenden Schritte umfasst:
a) Messen des Niveaus der gegen die 3S-Peptide der SEQ ID Nr. 2 gerichteten Antikörper in einer von dem Patienten entnommenen Probe,
b) Vergleichen des Niveaus der in Schritt a) gemessenen Anti-3S-Antikörper mit einem Referenzwert eines Anti-3S-Antikörper-Niveaus, welcher den Fortschritt der HIV-1-Erkrankung anzeigt.

2. Verfahren nach Anspruch 1, wobei Schritt a) durch die Immunodetektion der gegen das Peptid der SEQ ID Nr. 2 gerichteten Antikörper durchgeführt wird.

3. Verfahren nach Anspruch 1, wobei Schritt a) mit einer ELISA-Untersuchung durchgeführt wird, welche ein Polypeptid verwendet, welches das Peptid der SEQ ID Nr. 2 immobilisiert auf einem festen Träger enthält.

4. Verfahren nach Anspruch 1, ferner definiert als die folgenden Schritte umfassend:
1) Messen der viralen HIV-1-Last in der von dem Patienten entnommenen Probe, und
2) Vergleichen des Werts der viralen Last, der in Schritt 1) gemessen wurde, mit einem Referenzwert, der den Fortschritt der HIV-1-Erkrankung anzeigt.

5. In-vitro-Verfahren zur Prognose des Fortschritts einer HIV-1-Erkrankung nach Anspruch 1, wobei:
- Schritt a) ferner das Messen zumindest eines weiteren HIV-1-Erkrankungsprognose-Markers in der von dem HIV-1-infizierten Patienten entnommenen Probe enthält, und
- Schritt b) für jeden in Schritt a) gemessenen HIV-1-Erkrankungsprognose-Marker das Vergleichen des sich ergebenden Markerwerts mit einem Referenzwert für diesen Prognosemarker enthält.

6. In-vitro-Verfahren nach Anspruch 5, wobei der eine oder die mehreren weiteren HIV-1-Erkrankungsprognose-Marker aus der Gruppe ausgewählt wird, die besteht aus (i) der viralen HIV-Last, (ii) der absoluten Anzahl der CD4-T-Zellen, (iii) dem prozentualen Anteil der CD4-T-Zellen und (iv) dem Verhältnis der CD4+/CD8+ -T-Lymphozyten.

7. In-vitro-Verfahren nach Anspruch 6, wobei Schritt a) das Messen einer Kombination von HIV-1-Erkrankungsprognose-Markern enthält, welche ausgewählt sind aus der Gruppe bestehend aus:
- Anti-3S-Antikörper-Niveau kombiniert mit dem obigen Marker (i),
- Anti-3S-Antikörper-Niveau kombiniert mit dem obigen Marker (ii),
- Anti-3S-Antikörper-Niveau kombiniert mit dem obigen Marker (iii),
- Anti-3S-Antikörper-Niveau kombiniert mit dem obigen Marker (iv),
- Anti-3S-Antikörper-Niveau kombiniert mit den obigen Markern (i) und (ii),
- Anti-3S-Antikörper-Niveau kombiniert mit den obigen Markern (i) und (iii),
- Anti-3S-Antikörper-Niveau kombiniert mit den obigen Markern (i) und (iv),
- Anti-3S-Antikörper-Niveau kombiniert mit den obigen Markern (ii) und (iii),
- Anti-3S-Antikörper-Niveau kombiniert mit den obigen Markern (ii) und (iv),
- Anti-3S-Antikörper-Niveau kombiniert mit den obigen Markern (iii) und (iv),
- Anti-3S-Antikörper-Niveau kombiniert mit den obigen Markern (i), (ii) und (iii),
- Anti-3S-Antikörper-Niveau kombiniert mit den obigen Markern (i), (ii) und (iv),
- Anti-3S-Antikörper-Niveau kombiniert mit den obigen Markern (ii), (iii) und (iv),
- Anti-3S-Antikörper-Niveau kombiniert mit den obigen Markern (i), (iii) und (iv), und
- Anti-3S-Antikörper-Niveau kombiniert mit den obigen Markern (i), (ii), (iii) und (iv).

8. Verfahren zum Überwachen der Effizienz einer therapeutischen Behandlung, umfassend den Schritt der Durchführung des In-vitro-Verfahrens für die Prognose des Fortschritts einer HIV-1-Erkrankung nach einem der Ansprüche 1 bis 7 mit einer einem HIV-1-infizierten Patienten, der nicht zu dem seltenen Phänotyp eines asymptomatischen Langtermüberlebens gehört und der einer therapeutischen Behandlung mit einer pharmazeutischen Zusammensetzung unterzogen wird, die eine oder mehrere anti-retrovirale Wirkstoffe enthält, entnommenen Probe.

## Revendications

1. Méthode *in vitro* de pronostic de la progression d'une maladie associée à une infection par le VIH-1 chez un patient infecté avec un virus VIH-1, n'ayant pas le phénotype rare de survie des asymptomatiques à long terme, lequel pronostic est basé sur le taux d'anticorps dirigés contre le peptide 3S de SEQ ID N° 2 en tant que marqueur de pronostic, lequel marqueur de pronostic est indépendant du marqueur de pronostic de nombres de CD4, ladite méthode comprenant les étapes consistant à :
a) mesurer le taux d'anticorps dirigés contre le peptide 3S de SEQ ID N° 2 dans un échantillon recueilli auprès dudit patient,
b) comparer le taux d'anticorps anti-3S mesuré à l'étape a) à une valeur de référence du taux d'anticorps anti-3S révélatrice de la progression de la maladie associée à une infection par le VIH-1.

2. Méthode selon la revendication 1, dans laquelle l'étape a) est réalisée par immunodétection des anticorps dirigés contre le peptide de SEQ ID N° 2.

3. Méthode selon la revendication 1, dans laquelle l'étape a) est réalisée par dosage ELISA en utilisant un polypeptide comprenant le peptide de SEQ ID N° 2 immobilisé sur un support solide.

4. Méthode selon la revendication 1, définie en outre comme comprenant les étapes suivantes consistant à :
1) mesurer la charge virale du VIH-1 dans ledit échantillon recueilli auprès dudit patient, et
2) comparer la valeur de la charge virale mesurée à l'étape 1) avec une valeur de référence révélatrice de la progression de la maladie associée à une infection par le VIH-1.

5. Méthode *in vitro* de pronostic de la progression d'une maladie associée à une infection par le VIH-1 selon la revendication 1, dans laquelle :
- l'étape a) comprend en outre la mesure d'au moins un autre marqueur de pronostic de la maladie associée à une infection par le VIH-1 dans un échantillon recueilli auprès d'un patient infecté avec le VIH-1, et
- l'étape b) comprend la comparaison, pour chaque marqueur de pronostic de la maladie associée à une infection par le VIH-1 mesuré à l'étape a), de la valeur résultante du marqueur avec une valeur de référence pour ledit marqueur de pronostic.

6. Méthode *in vitro* selon la revendication 5, dans laquelle ledit ou lesdits autres marqueurs de pronostic de la maladie associée à une infection par le VIH-1 sont choisis dans le groupe constitué par (i) la charge virale du VIH, (ii) le nombre absolu de lymphocytes T CD4, (iii) le pourcentage de lymphocytes T CD4 et (iv) le rapport de lymphocytes T CD4+/CD8+.

7. Méthode *in vitro* selon la revendication 6, dans laquelle l'étape a) comprend la mesure d'une combinaison de marqueurs de pronostic de la maladie associée à une infection par le VIH-1 choisis dans le groupe constitué par :
- le taux d'anticorps anti-3S combiné avec le marqueur (i) ci-dessus,
- le taux d'anticorps anti-3S combiné avec le marqueur (ii) ci-dessus,
- le taux d'anticorps anti-3S combiné avec le marqueur (iii) ci-dessus,
- le taux d'anticorps anti-3S combiné avec le marqueur (iv) ci-dessus,
- le taux d'anticorps anti-3S combiné avec les marqueurs (i) et (ii) ci-dessus,
- le taux d'anticorps anti-3S combiné avec les marqueurs (i) et (iii) ci-dessus,
- le taux d'anticorps anti-3S combiné avec les marqueurs (i) et (iv) ci-dessus,
- le taux d'anticorps anti-3S combiné avec les marqueurs (ii) et (iii) ci-dessus,
- le taux d'anticorps anti-3S combiné avec les marqueurs (ii) et (iv) ci-dessus,
- le taux d'anticorps anti-3S combiné avec les marqueurs (iii) et (iv) ci-dessus,
- le taux d'anticorps anti-3S combiné avec les marqueurs (i), (ii) et (iii) ci-dessus,
- le taux d'anticorps anti-3S combiné avec les marqueurs (i), (ii) et (iv) ci-dessus,
- le taux d'anticorps anti-3S combiné avec les marqueurs (ii), (iii) et (iv) ci-dessus,
- le taux d'anticorps anti-3S combiné avec les marqueurs (i), (iii) et (iv) ci-dessus, et
- le taux d'anticorps anti-3S combiné avec les marqueurs (i), (ii), (iii) et (iv) ci-dessus.

8. Méthode de surveillance de l'efficacité d'un traitement thérapeutique comprenant l'étape consistant à réaliser la méthode *in vitro* pour le pronostic de la progression d'une maladie associée à une infection par le VIH-1 selon l'une quelconque des revendications 1 à 7 sur un échantillon recueilli auprès d'un patient infecté avec le VIH-1 qui n'a pas le phénotype rare de survie des asymptomatiques à long terme et qui reçoit un traitement thérapeutique avec une composition pharmaceutique comprenant un ou plusieurs agents antirétroviraux.


### SEQUENCE LISTING

<110> InnaVirVax Institut National de la Sante et de la Recherche Medicale
<120> A Method for the prognosis of progression of the HIV disease
<130> BR95301
<150> EP 11305187.4 <151> 2011-02-22
<160> 4
<170> PatentIn version 3.5
<210> 1
   <211> 6
   <212> PRT
   <213> Human immunodeficiency virus
<400> 1
   Ser Trp Ser Asn Lys Ser
   1               5
<210> 2
   <211> 16
   <212> PRT
   <213> Human immunodeficiency virus
<400> 2
   Cys Pro Trp Asn Ala Ser Trp Ser Asn Lys Ser Leu Asp Asp Ile Trp
   1               5                   10                  15
<210> 3
   <211> 6
   <212> PRT
   <213> Human immunodeficiency virus
<400> 3
   Ser Trp Ser Asn Lys Thr
   1               5
<210> 4
   <211> 6
   <212> PRT
   <213> Human immunodeficiency virus
<400> 4
   Ser Trp Ser Asn Arg Ser
   1               5